# EUROPÄISCHE PATENTANMELDUNG

(11) **EP 3 574 948 A1**
(43) Veröffentlichungstag der Anmeldung: **04.12.2019**
(21) Anmeldenummer: 18175549.7
(22) Anmeldetag: 01.06.2018
(51) Int. Cl.: A61M 11/02, A61M 15/00, A61M 11/04, A61M 16/10, H05B 3/00, A24F 47/00, A61L 9/00

(54) **VORRICHTUNG ZUM HERSTELLEN VON FÜR EINE INHALATION BESTIMMTER LUFT UND HEIZUNG FÜR EINE VORRICHTUNG ZUM HERSTELLEN VON FÜR EINE INHALATION BESTIMMTER LUFT**

(71) Anmelder: Jencke, Lutz, 30165 Hannover (DE); Kip, Ralf, 30167 Hannover (DE)
(72) Erfinder: Jencke, Lutz, 30165 Hannover (DE); Kip, Ralf, 30167 Hannover (DE)
(74) Vertreter: Jabbusch, Matthias

(57) **Zusammenfassung**

Bei einer Vorrichtung zum Herstellen von für eine Inhalation bestimmter Luft, umfassend zumindest ein einen Luftweg durch die Vorrichtung bereitstellendes Luftleitungssystem mit Luftleitungen (7) sowie eine dem Luftleitungssystem zugeordnete Heizung (9) für die Luft, wobei wenigstens eine in den Luftweg einbringbare Aufnahmeeinrichtung (3) für Partikel vorgesehen ist, ist vorgesehen, dass das Luftleitungssystem in einem Bereich oberhalb der Aufnahmeeinrichtung für die Partikel unterbrochen ist und beiden freien Luftleitungsenden im Bereich dieser Unterbrechung Dichtungen (13) zugeordnet sind, wobei für die Aufnahmeeinrichtung eine Hebeeinrichtung (14) zu ihrem Anheben gegen die Dichtungen der Luftleitungsenden vorgesehen ist

Eine Heizung für eine vorgenannte Vorrichtung zum Herstellen von für eine Inhalation bestimmter Luft, umfassend zumindest ein einem Abschnitt eines Luftleitungssystems zugeordnetes Heizelement (22), zeichnet sich dadurch aus, dass das Heizelement von außen auf den Luftleitungsabschnitt aufgelegt ist und dass im Inneren des Luftleitungsabschnitts dreidimensionale Körper (24) eingebracht sind.

## Beschreibung

Die Erfindung betrifft eine Vorrichtung zum Herstellen von für eine Inhalation bestimmter Luft, umfassend zumindest ein einen Luftweg durch die Vorrichtung bereitstellendes Luftleitungssystem mit Luftleitungen sowie eine dem Luftleitungssystem zugeordnete Heizung für die Luft, wobei wenigstens eine in den Luftweg einbringbare Aufnahmeeinrichtung für Partikel vorgesehen ist. Die Erfindung betrifft des Weiteren eine Heizung für eine Vorrichtung zum Herstellen von für eine Inhalation bestimmter Luft, insbesondere für eine vorgenannte Vorrichtung, umfassend zumindest ein einem Abschnitt eines Luftleitungssystems zugeordnetes Heizelement.

Bei Atemwegsbeschwerden oder auch bei Erkrankungen insbesondere der Atemwege ist eine Therapieform das Inhalieren von Luft. Die Luft wird dazu mit Wirkstoffen aus Partikeln, wie Kräutern, angereichert, die bei Auftreffen auf z. B. entzündete Bereiche der Atemwege Heilung bringen.

Vorrichtungen zum Herstellen von für eine Inhalation bestimmter Luft nach dem Stand der Technik sind bekannt. Während als Hausmittel ein über den Kopf gezogenes Handtuch über einer mit warmer Flüssigkeit gefüllten Schüssel fungiert, werden in industriell hergestellten Vorrichtungen Heizungen eingesetzt, um die zu inhalierende Luft zu erwärmen. Die zu inhalierende Luft wird regelmäßig über Partikel geführt, um in den Partikeln enthaltene Wirkstoffe aus den Partikeln zu entnehmen und der Inhalationsluft zuzuführen.

Der Erfindung liegt die Aufgabe zugrunde, eine Vorrichtung der eingangs Gattung aufzuzeigen, mit der ein möglichst verlustfreier Eintritt der in den Partikeln enthaltenen Wirkstoffe in die Luft bei einfacher Handhabung der Partikel ermöglicht ist. Weiterhin soll eine Heizung aufgezeigt werden, mit der ein vollständiges und gleichmäßiges Erwärmen der Luft zu erzielen ist.

Hinsichtlich der Vorrichtung ist diese Aufgabe erfindungsgemäß dadurch gelöst, dass das Luftleitungssystem in einem Bereich oberhalb der Aufnahmeeinrichtung für die Partikel unterbrochen ist und beiden freien Luftleitungsenden im Bereich dieser Unterbrechung Dichtungen zugeordnet sind, wobei für die Aufnahmeeinrichtung eine Hebeeinrichtung zu ihrem Anheben gegen die Dichtungen der Luftleitungsenden vorgesehen ist.

Durch das Luftleitungssystem wird die Luft für die Inhalation geführt. Diese Luft ist nach Passieren der Heizung erwärmt und ist nun mit den Partikeln in Kontakt zu bringen, damit die Wirkstoffe aus den Partikeln in die Luft übergehen. Dafür ist nach der Erfindung vorgesehen, das Luftleitungssystem in seinem Verlauf zu unterbrechen. So ist erreicht, dass aufgrund dieser Unterbrechung zwei freie Lüftungsenden gegeben sind. Aus einem Lüftungsende kann die erwärmte Luft ausströmen, in das andere Lüftungsende ist die dann mit den Wirkstoffen der Partikel angereicherte Luft einzuführen. Diesen Lüftungsenden werden Dichtungen zugeordnet.

Die Führung der Luft im Bereich der Unterbrechung des Luftleitungssystems erfolgt mit der Aufnahmeeinrichtung für die Partikel. Die Luft wird in diese Aufnahmeeinrichtung eingeführt und trifft hier auf die zuvor eingebrachten Partikel. Anschließend kann die Luft aus der Aufnahmeeinrichtung zurück in das andere Luftleitungsende eintreten und einer Inhalation zugeführt werden.

Damit bei dieser Luftleitung keine Verluste insbesondere der mit den Wirkstoffen angereicherten Luft eintritt, sind zum einen die Dichtungen an den freien Luftleitungsenden vorgesehen und ist zum anderen die Hebeeinrichtung zum Anheben der Aufnahmeeinrichtung gegen die Dichtungen der Luftleitungsenden vorgesehen. Die Aufnahmeeinrichtung wird mit der Hebeeinrichtung gegen die Dichtungen der Luftleitungsenden geführt, sie kann sich an diese Dichtungen anlegen und insofern einen dichten Weg für die geführte Luft ausbilden.

Nach einer ersten Weiterbildung der Erfindung ist vorgesehen, dass die Luftleitungen oberhalb der Aufnahmeeinrichtung für die Partikel parallel zueinander und vertikal ausgerichtet sind. Bei dieser Anordnung wird die erwärmte Luft von oben in die Aufnahmeeinrichtung eingeführt, sie wird an der Aufnahmeeinrichtung umgelenkt und wieder nach oben weggeführt. Dafür ist es zweckmäßig, dass die freien Enden der Luftleitungen auf einer Höhe angeordnet sind, so dass bei einem Anheben der Aufnahmeeinrichtung eine etwa zeitgleiche Anlage der Aufnahmeeinrichtung an den Dichtungen der Luftleitungsenden erfolgt. Die Dichtungen sind dabei vorzugsweise aus Silikon gefertigt. Für die Dichtungen sind Aufnahmen aus einem Kunststoff vorgesehen, beispielsweise aus Polyetheretherketon (PEEK). Silikon und PEEK sind Materialien, die hitzebeständig und in Bezug auf Silikon lebensmitteltauglich sind.

Nach einer nächsten Weiterbildung der Erfindung ist vorgesehen, dass die Aufnahmeeinrichtung als Schublade ausgebildet ist. Die Ausbildung einer Schublade ermöglicht eine einfache Handhabung beim Einführen von Partikeln in die Vorrichtung. Die Partikel können in die Schublade gegeben werden und ihre Wirkstoffe an in die Schublade geführte Luft abgeben. Als Material für die Schublade kann beispielsweise Edelstahl vorgesehen sein.

Nach einer nächsten Weiterbildung der Erfindung ist vorgesehen, dass die Schublade eine Einhängeeinrichtung für einen Siebeinsatz für die Partikel hat. Ein Siebeinsatz für Partikel kann gehandhabt werden wie ein Teesieb. Partikel können in den Siebeinsatz eingegeben werden und dann in die Einhängeeinrichtung der Schublade gehängt werden. Das erfolgt beispielsweise dann, wenn die Schublade nach außen in eine Einfüllposition gezogen wurde. Nach Einlegen des Siebeinsatzes für die Partikel mit den Partikeln kann die Schublade wieder in die andere Richtung verschoben werden und in die Vorrichtung eingeführt werden. Dort wird sie zumindest abschnittsweise dann gegen die Dichtungen der Luftleitungsenden gehoben.

Für dieses Führen der Schublade sieht eine Weiterbildung der Erfindung vor, dass die Hebeeinrichtung einen federunterstützten Kurbeltrieb aufweist. Mit einem Kurbeltrieb kann das Anheben der Schublade in eine höhere oder in eine niedere Position durchgeführt werden. In der höheren Position wird die Schublade gegen die Dichtungen der Luftleitungsenden geführt. In der unteren Position ist sie vorzugsweise für ein Herausziehen aus der Vorrichtung zum Nachfüllen von Partikeln ausgebildet. Die Federunterstützung führt zumindest einen Abschnitt der Schublade in die obere Hubposition gegen die Dichtungen. Eine enge Anlage der Schublade an den Dichtungen der Luftleitungsenden ist damit gewährleistet, so dass es keine Luftleitungsverluste während der Passage der Luft an den Partikeln gibt.

Zur weiteren Ausbildung der erfindungsgemäßen Vorrichtung kann noch vorgesehen sein, dass das Luftleitungssystem in zumindest ein Inhalationsstück mündet. Die Luft, die die Partikel passiert hat, kann somit direkt für eine Inhalation verwendet werden. Ein Patient kann über das Inhalationsstück diese Luft aufnehmen. Der Transport der Luft durch das Luftleitungssystem erfolgt dann vorzugsweise durch den Patienten selber, in dem er durch ein Einatmen einen Unterdruck bereitstellt.

Alternativ oder zusätzlich kann vorgesehen sein, dass das Luftleitungssystem in einen Stutzen mündet, wobei dem Luftleitungssystem zumindest eine Luftpumpe zugeordnet ist. Bei dem Führen der Luft zu einem Stutzen fehlt ein durch einen Patienten bereitgestelltes Vakuum. Hier ist eine Luftpumpe vorgesehen, die die Luft zum Stutzen führt. Die Hebeeinrichtung für die Schublade ist dabei mit einem Schalter ausgerüstet, der die Luftpumpe stilllegt, wenn die Hebeeinrichtung abgesenkt wird. Damit ist ein Verwirbeln und ein Austritt von Partikeln aus dem Siebeinsatz verhindert.

An den Stutzen kann ein Behälter zur Aufnahme der Luft angeschlossen werden, beispielsweise ein Ballon.

Die erfindungsgemäße Lösung der auf die Heizung bezogenen Aufgabe zeichnet sich dadurch aus, dass das Heizelement von außen auf einen Luftleitungsabschnitt für die Luft aufgelegt ist und dass im Inneren des Luftleitungsabschnitts dreidimensionale Körper eingebracht sind.

Die erfindungsgemäße Heizung, für das selbständiger Schutz beansprucht wird, fügt sich in das Luftleitungssystem ein. Zumeist ein Heizelement legt sich dafür von außen auf einen Luftleitungsabschnitt auf. Damit ist sichergestellt, dass sämtliche Luft das Heizelement passiert. Das Heizen der Luft erfolgt dann mit dem Heizelement. Wärme wird somit außerhalb des Luftleitungsabschnitts erzeugt, dies aber in unmittelbarer Zuordnung zum Luftleitungsabschnitt. Die Wärme kann durch den Luftleitungsabschnitt hindurchtreten und auf die durch den Luftleitungsabschnitt geführte Luft einwirken und diese und die eingebrachten Körper erwärmen.

Wärme wird somit von außen eingetragen, die erfindungsgemäß im Inneren des Luftleitungsabschnitts angeordneten Körper führen die Wärme von außen auch nach innen. Die Luft wird zudem an den eingebrachten Körper abgelenkt. Eine laminare Strömung durch den Leitungsabschnitt ist durch die Körper unterbrochen, die Luft wird nach innen, nach außen abgelenkt und passiert dabei die Körper. Die Körper erwärmen sich, da sie innerhalb des Luftleitungsabschnittes angeordnet sind, sie geben gleichfalls Wärme an die Luft ab. Es findet eine Oberflächenvergrößerung durch die Körper statt, so dass über eine größere Fläche Wärme in die Luft eingetragen wird. Mit Vorteil erfolgt eine vollständige und gleichmäßige Erwärmung der Luft über den gesamten Querschnitt der Luftleitung.

Nach einer Weiterbildung der Erfindung kann vorgesehen sein, dass die Körper miteinander verklebt sind. Als Kleber kann beispielsweise ein keramischer Kleber dienen. Dieser ist aus einem wärmeleitfähigen Material ausgebildet.

Das Heizelement ist dabei vorzugsweise als Flächenelement ausgebildet und flächig auf den Luftleitungsabschnitt aufgelegt. Es können mehrere Heizelemente vorgesehen sein, die voneinander verschiedene Abschnitte des Luftleitungsabschnitts bedecken. Dabei können Anschlüsse für 230 V oder separate Anschlüsse für 115 V eingerichtet sein.

Hinsichtlich der in den Luftleitungsabschnitt eingebrachten Körper sieht eine Weiterbildung der Heizung vor, dass die Körper Kugeln sind. Kugeln haben eine jeweils abgerundete Oberfläche, an der Luft abgelenkt wird. Dabei kann insbesondere eine Schüttung von Kugeln mit voneinander verschiedenen Durchmessern vorgesehen sein. Es können auch andere Körper vorgesehen sein, beispielsweise Füllkörper als Pellets, Raschigringe, Sattelkörper oder Schrot.

Für die Kugeln werden bevorzugt wärmeleitfähige Materialien eingesetzt, beispielsweise Keramik oder ein Metall.

Ein Verkleben von Kugeln mit dem keramischen Kleber vergrößert die Kontaktfläche zweier einander benachbarter Kugeln. Damit ist eine bessere Wärmeleitung von einer Kugel in die benachbarte Kugel ermöglicht.

Ein Ausführungsbeispiel der Erfindung ist in der Zeichnung dargestellt. Es zeigen:
- Figur 1:: eine perspektivische Ansicht einer erfindungsgemäßen Vorrichtung zum Herstellen von für eine Inhalation bestimmter Luft,
- Figur 2:: eine weitere perspektivische Ansicht der Vorrichtung nach Figur 1 mit verkleinertem Maßstab,
- Figur 3:: eine Schnittansicht der Vorrichtung gemäß Figur 1 und
- Figur 4:: eine Teilansicht von Bauteilen der Vorrichtung gemäß Figur 1,
- Figur 5:: eine perspektivische Ansicht einer Heizung für eine Vorrichtung gemäß Figur 1 und
- Figur 6:: eine Schnittansicht der Heizung gemäß Figur 5.

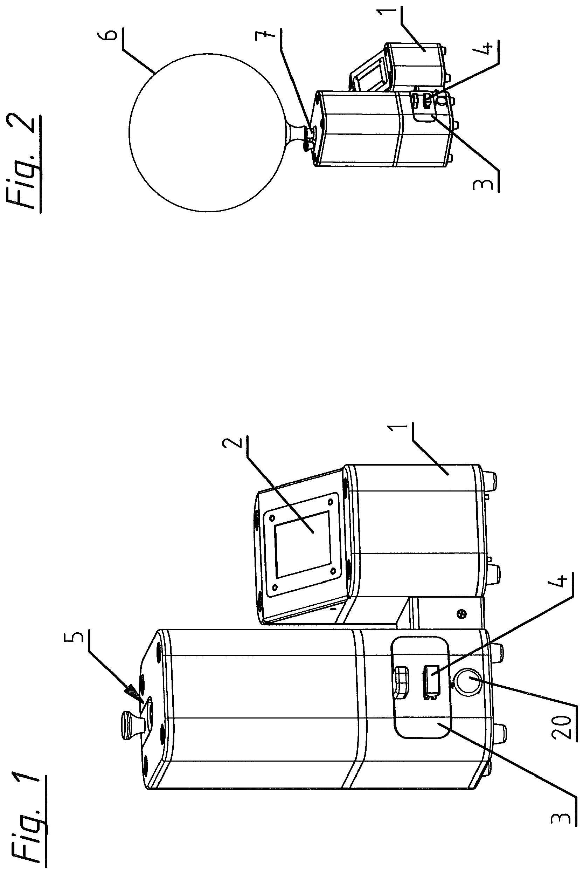

Die Vorrichtung in Figur 1 hat ein Gehäuse 1 und ein Bedienungsdisplay 2. Im linken höheren Abschnitt des Gehäuses 1 ist eine Schublade 3 mit einem Handgriff 4 angeordnet. Auf der Oberseite des linken Abschnitts des Gehäuses 1 ist ein Anschluss 5 für einen Ballon 6 gegeben, wie in Figur 2 gezeigt. In den Anschluss 5 wird ein Stutzen 7 gebracht, auf den der Ballon 6 aufgesteckt wird. Mit der Vorrichtung hergestellte Luft, die für eine Inhalation bestimmt ist, kann in dem Ballon 6 für eine spätere Verwendung aufbewahrt werden.

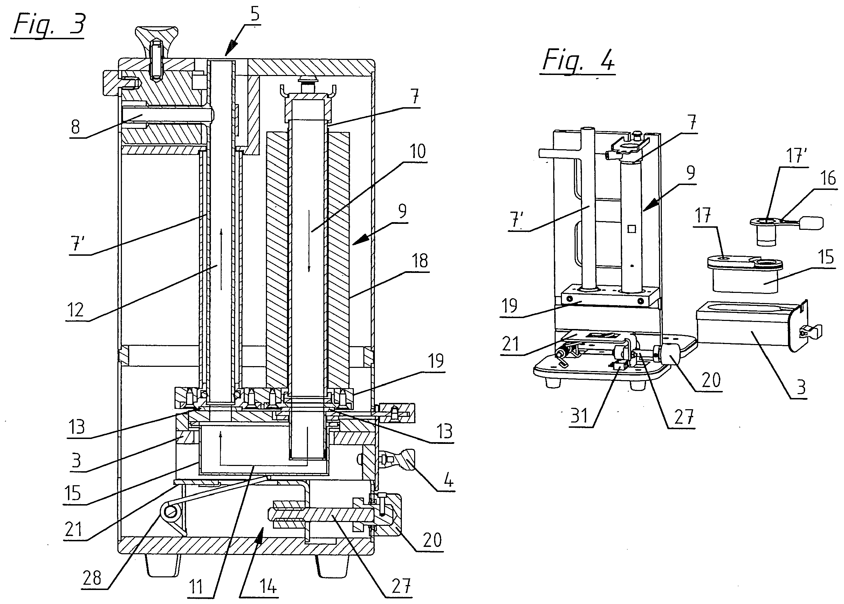

Figur 3 zeigt, dass innerhalb der Vorrichtung ein Luftführungssystem enthalten ist. Dieses Luftführungssystem umfasst Luftleitungen 7, 7' die parallel zueinander und vertikal ausgerichtet sind. Die Luftleitung 7' verläuft zu dem Anschluss 5 für den Ballon 6. Die Luftleitung 7' ist mit einer Abzweigungsleitung 8 luftleitend verbunden. An die Abzweigungsleitung 8 kann ein nicht weiter dargestelltes Inhalationsstück angeschlossen sein.

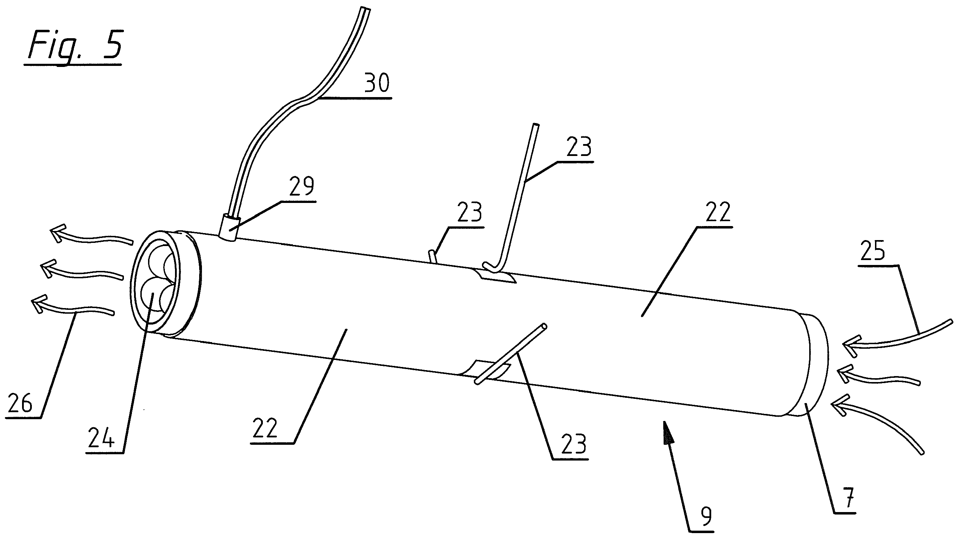

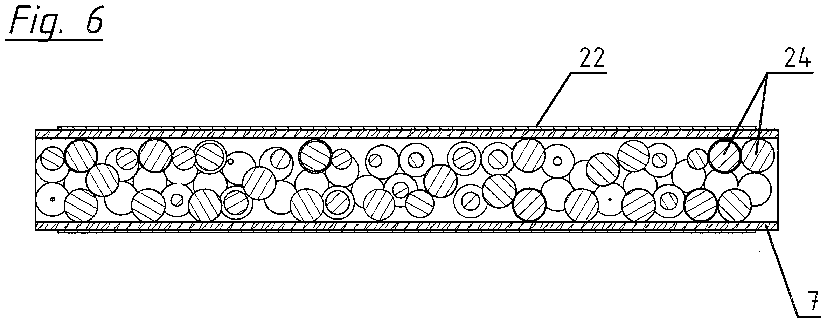

Der Luftleitung 7 ist eine Heizung 9 für durch die Luftleitung 7 geführter Luft zugeordnet, siehe Figuren 5 und 6.

Die Luft wird durch die Luftleitung 7 entlang Pfeil 10 geführt und tritt am unteren Ende der Luftleitung 7 in die Schublade 3 ein. Durch diese Schublade 3 wird die Luft entlang Pfeil 11 in die Luftleitung 7' geführt und in dieser nach oben geleitet (Pfeil 12). Mit einem auf der Oberseite der Vorrichtung angeordneten Griff kann die Luftleitung 7' aus der Vorrichtung entnommen werden. Die seitlich vorgesehene Rändelschraube wird dazu entfernt, nach der Entnahme der Luftleitung 7' ist diese einer Reinigung zuführbar.

Beide Luftleitungen 7, 7' sind an ihrem unteren freien Ende jeweils mit einer Dichtung 13 ausgerüstet. Gegen diese Dichtungen 13 wird die Schublade 3 mit einer Hebeeinrichtung 14 angehoben. Die Hebeeinrichtung 14 besteht dabei aus einem Kurbeltrieb 27 und einer Feder 28. In der in Figur 3 gezeigten oberen, angehobenen Stellung der Schublade 3 wird die Schublade 3 mit der Feder 28 gegen die Dichtungen 13 der Luftleitungen 7, 7' geführt.

Figur 4 zeigt den Aufbau der Schublade 3. Die Schublade 3 hat einen Einsatz 15, der einen Siebeinsatz 16 für Partikel aufnimmt. Der Einsatz 15 ist so ausgebildet, dass die Schublade 3 an ihrer Oberseite weitgehend geschlossen ist und lediglich zwei Öffnungen 17, 17' in Einsatz 15 und Siebeinsatz 16 gegeben sind, die mit der Hebeeinrichtung 14 gegen die Dichtungen 13 der Luftleitungen 7, 7' geführt werden. Die Luft tritt durch die Öffnung 17' zunächst in die Siebeinsatz 16 ein und passiert hier in den Siebeinsatz 16 eingebrachte Partikel. Die Luft wird dann weiter durch den Einsatz 15 geführt und tritt durch die Öffnung 17 wieder aus dem Einsatz 15 aus. Sie tritt anschließend in die Luftleitung 7' ein. Die Luftleitung 7 hat dabei eine Wärmeisolation 18 (Figur 1).

Figur 4 zeigt noch, dass die Luftleitungen 7, 7' auf einer Konsole 19 aufstehen. Damit sind die unteren freien Enden der Luftleitungen 7, 7' auf einer Höhe angeordnet. Der Kurbeltrieb 27 hat einen von außen bedienbaren Drehknopf 20. Der Kurbeltrieb 27 wirkt dabei auf eine Schiebeführung 21 für die eine Feder 28. Diese Schiebeführung 21 ist durchbrochen, die Feder 28 greift durch den Durchbruch. Ein Anheben des Einsatzes 15 der Schublade 3 bewirkt das Führen des Einsatzes 15 gegen die Dichtungen 13. Figur 4 zeigt noch einen Schalter 31. Dieser Schalter 31 registriert ein Absenken des Einsatzes 15 der Schublade 3. Tritt dieses Absenken ein, wird eine Pumpe für die Luftführung durch die Luftleitungen 7, 7' stillgelegt.

Figur 4 zeigt auch die Heizung 9, die der Luftleitung 7 zugeordnet ist. Die Heizung 9 umfasst in Figur 5 dargestellte Heizelemente 22, die von außen auf die Luftleitung 7 als Abschnitt eines Luftleitungssystems aufgelegt sind. Elektrische Zuleitungen 23 versorgen die Heizelemente 22 mit elektrischer Energie. Im Inneren der Luftleitung 7 sind im Bereich der Heizelemente 22 Kugeln 24 angeordnet. Die zu erwärmende Luft tritt in die Heizung 9 entlang der Pfeile 25 ein. Die erwärmte Luft verlässt die Heizung 9 entlang der Pfeile 26. Der Heizung 9 ist dabei noch ein Temperatursensor 29 aufgelegt zugeordnet. Dieser Temperatursensor hat seine eigene Stromversorgung über eine Zuleitung 30.

Figur 6 zeigt, dass die Kugeln 24 im Inneren der Luftleitung 7 in einer Schüttung vorliegen. Es sind Kugeln 24 vorhanden, die Schüttung erfolgt eng, dabei berühren Kugeln 24 auch die Innenfläche der Luftleitung 7. Eintretende Luft wird durch die Kugeln 24 abgelenkt und wird auch gegen die Innenseite der Luftleitung 7 geführt und erwärmt.

## Patentansprüche

1. Vorrichtung zum Herstellen von für eine Inhalation bestimmter Luft, umfassend zumindest ein einen Luftweg durch die Vorrichtung bereitstellendes Luftleitungssystem mit Luftleitungen sowie eine dem Luftleitungssystem zugeordnete Heizung für die Luft, wobei wenigstens eine in den Luftweg einbringbare Aufnahmeeinrichtung für Partikel vorgesehen ist,
**dadurch gekennzeichnet,**
**dass** das Luftleitungssystem in einem Bereich oberhalb der Aufnahmeeinrichtung für die Partikel unterbrochen ist und beiden freien Luftleitungsenden im Bereich dieser Unterbrechung Dichtungen (13) zugeordnet sind, wobei für die Aufnahmeeinrichtung eine Hebeeinrichtung (14) zu ihrem Anheben gegen die Dichtungen (13) der Luftleitungsenden vorgesehen ist.

2. Vorrichtung nach Anspruch 1, **dadurch gekennzeichnet, dass** die Luftleitungen (7, 7') oberhalb der Aufnahmeeinrichtung für die Partikel parallel zueinander und vertikal ausgerichtet sind.

3. Vorrichtung nach Anspruch 1 oder 2, **dadurch gekennzeichnet, dass** die freien Enden der Luftleitungen (7, 7') auf einer Höhe angeordnet sind.

4. Vorrichtung nach einem der vorhergehenden Ansprüche, **dadurch gekennzeichnet, dass** die Aufnahmeeinrichtung als Schublade (3) ausgebildet ist.

5. Vorrichtung nach Anspruch 4, **dadurch gekennzeichnet, dass** die Schublade (3) eine Einhängeeinrichtung für einen Siebeinsatz (16) für die Partikel hat.

6. Vorrichtung nach einem der vorhergehenden Ansprüche, **dadurch gekennzeichnet, dass** die Hebeeinrichtung (14) einen federunterstützten Kurbeltrieb (27) aufweist.

7. Vorrichtung nach einem der vorhergehenden Ansprüche, **dadurch gekennzeichnet, dass** das Luftleitungssystem in zumindest ein Inhalationsstück mündet.

8. Vorrichtung nach einem der vorhergehenden Ansprüche, **dadurch gekennzeichnet, dass** das Luftleitungssystem in einen Stutzen mündet, wobei dem Luftleitungssystem zumindest eine Luftpumpe zugeordnet ist.

9. Heizung für eine Vorrichtung zum Herstellen von für eine Inhalation bestimmter Luft, insbesondere für eine Vorrichtung nach zumindest einem der Ansprüche 1 bis 8, umfassend zumindest ein einem Abschnitt eines Luftleitungssystems zugeordnetes Heizelement,
**dadurch gekennzeichnet,**
**dass** das Heizelement (22) von außen auf den Luftleitungsabschnitt aufgelegt ist und dass im Inneren des Luftleitungsabschnitts dreidimensionale Körper eingebracht sind.

10. Heizung nach Anspruch 9, **dadurch gekennzeichnet, dass** das Heizelement (22) als Flächenelement ausgebildet ist und flächig auf den Luftleitungsabschnitt aufgelegt ist.

11. Heizung nach Anspruch 9 oder 10, **dadurch gekennzeichnet, dass** die Körper Kugeln (24) sind.

12. Heizung nach Anspruch 11, **dadurch gekennzeichnet, dass** im Inneren des Luftleitungsabschnitts eine Schüttung von Kugeln (24) angeordnet ist.

13. Heizung nach einem der Ansprüche 9 bis 12, **dadurch gekennzeichnet, dass** die Körper aus Keramik gefertigt sind.

14. Heizung nach einem der Ansprüche 9 bis 13, **dadurch gekennzeichnet, dass** die Körper miteinander verklebt sind.

15. Heizung nach Anspruch 14, **dadurch gekennzeichnet, dass** der Kleber für die Körper ein keramischer Kleber ist.

## Geänderte Patentansprüche

### Geänderte Patentansprüche gemäss Regel 137(2) EPÜ.

1. Vorrichtung zum Herstellen von für eine Inhalation bestimmter Luft, umfassend zumindest ein einen Luftweg durch die Vorrichtung bereitstellendes Luftleitungssystem mit Luftleitungen sowie eine dem Luftleitungssystem zugeordnete Heizung für die Luft, wobei wenigstens eine in den Luftweg einbringbare Aufnahmeeinrichtung für Partikel vorgesehen ist,
**dadurch gekennzeichnet,**
**dass** das Luftleitungssystem in einem Bereich oberhalb der Aufnahmeeinrichtung für die Partikel unterbrochen ist und beiden freien Luftleitungsenden im Bereich dieser Unterbrechung Dichtungen (13) zugeordnet sind, wobei für die Aufnahmeeinrichtung eine Hebeeinrichtung (14) zu ihrem Anheben gegen die Dichtungen (13) der Luftleitungsenden vorgesehen ist.

2. Vorrichtung nach Anspruch 1, **dadurch gekennzeichnet, dass** die Luftleitungen (7, 7') oberhalb der Aufnahmeeinrichtung für die Partikel parallel zueinander und vertikal ausgerichtet sind.

3. Vorrichtung nach Anspruch 1 oder 2, **dadurch gekennzeichnet, dass** die freien Enden der Luftleitungen (7, 7') auf einer Höhe angeordnet sind.

4. Vorrichtung nach einem der vorhergehenden Ansprüche, **dadurch gekennzeichnet, dass** die Aufnahmeeinrichtung als Schublade (3) ausgebildet ist.

5. Vorrichtung nach Anspruch 4, **dadurch gekennzeichnet, dass** die Schublade (3) eine Einhängeeinrichtung für einen Siebeinsatz (16) für die Partikel hat.

6. Vorrichtung nach einem der vorhergehenden Ansprüche, **dadurch gekennzeichnet, dass** die Hebeeinrichtung (14) einen federunterstützten Kurbeltrieb (27) aufweist.

7. Vorrichtung nach einem der vorhergehenden Ansprüche, **dadurch gekennzeichnet, dass** das Luftleitungssystem in zumindest ein Inhalationsstück mündet.

8. Vorrichtung nach einem der vorhergehenden Ansprüche, **dadurch gekennzeichnet, dass** das Luftleitungssystem in einen Stutzen mündet, wobei dem Luftleitungssystem zumindest eine Luftpumpe zugeordnet ist.

9. Heizung für eine Vorrichtung zum Herstellen von für eine Inhalation bestimmter Luft nach zumindest einem der Ansprüche 1 bis 8, umfassend zumindest ein einem Abschnitt eines Luftleitungssystems zugeordnetes Heizelement,
**dadurch gekennzeichnet,**
**dass** das Heizelement (22) von außen auf den Luftleitungsabschnitt aufgelegt ist und dass im Inneren des Luftleitungsabschnitts dreidimensionale Körper eingebracht sind.

10. Heizung nach Anspruch 9, **dadurch gekennzeichnet, dass** das Heizelement (22) als Flächenelement ausgebildet ist und flächig auf den Luftleitungsabschnitt aufgelegt ist.

11. Heizung nach Anspruch 9 oder 10, **dadurch gekennzeichnet, dass** die Körper Kugeln (24) sind.

12. Heizung nach Anspruch 11, **dadurch gekennzeichnet, dass** im Inneren des Luftleitungsabschnitts eine Schüttung von Kugeln (24) angeordnet ist.

13. Heizung nach einem der Ansprüche 9 bis 12, **dadurch gekennzeichnet, dass** die Körper aus Keramik gefertigt sind.

14. Heizung nach einem der Ansprüche 9 bis 13, **dadurch gekennzeichnet, dass** die Körper miteinander verklebt sind.

15. Heizung nach Anspruch 14, **dadurch gekennzeichnet, dass** der Kleber für die Körper ein keramischer Kleber ist.
